# EUROPEAN PATENT SPECIFICATION

(11) **EP 1 673 018 B1**
(45) Date of publication and mention of the grant of the patent: **07.11.2007**
(21) Application number: 04783714.1
(22) Date of filing: 09.09.2004
(51) Int. Cl.: A61B 17/12

(54) **ENERGY ACTIVATED VASO-OCCLUSIVE DEVICES**
ENERGIEAKTIVIERTE VASOOKKLUSIVE VORRICHTUNGEN
DISPOSITIFS VASO-OCCLUSIFS ACTIVES PAR ENERGIE

(30) Priority: 23.09.2003 US 669203
(43) Date of publication of application: 28.06.2006
(73) Proprietor: Boston Scientific Limited, Christ Church (BB)
(72) Inventor: WALLACE, Michael, P., Pleasanton, CA 94566 (US)
(74) Representative: Lecomte, Didier
(86) International application number: PCT/US2004/029589
(87) International publication number: WO 2005/032380

(56) References cited:
- WO-A-02/087416
- US-A- 5 749 894
- US-A- 5 853 418
- US-A1- 2002 138 134
- US-B1- 6 231 590
- PATENT ABSTRACTS OF JAPAN vol. 1995, no. 04, 31 May 1995 (1995-05-31) -& JP 07 018357 A (YASUBUMI FURUYA; others: 01), 20 January 1995 (1995-01-20)

## Description

### FIELD OF INVENTION

The invention relates generally to vaso-occlusive devices comprising a material which may be heated by application of a source of energy (e.g., electro-magnetic energy) external to a patient's body after the device is implanted at a treatment site.

### BACKGROUND

Vaso-occlusive devices are implants that are placed in cavities, e.g., an aneurysm, blood vessel lumen, fistula, or other cavity in a patient's vasculature for the purpose of facilitating formation of an thrombus or embolism. Such vaso-occlusive devices are typically delivered by a catheter that is advanced to a treatment site endoluminally, e.g., from a percutaneous entry site, using conventional access procedures.

Well known vaso-occlusive devices include helically-wound coils that assume an elongate, "delivery" configuration when constrained within a delivery catheter, and a three-dimensional, "deployed" configuration when deployed in the body cavity and no longer constrained in the catheter. Once deployed, such vaso-occlusive devices help promote embolization and/or occlusion of the cavity. For example, vaso-occlusive devices are used to fill aneurysm cavities to reduce the risk of the further growth and/or rupture of the aneurysm.

To enhance embolization, it has been suggested to provide a coating on vaso-occlusive devices that reacts in some beneficial way (e.g., with blood) in the body. For example, U.S. Patent No. 6,187,024 discloses vaso-occlusive devices coated with a bioactive agent and/or collagenous material. It has also been suggested to provide a coating on a vaso-occlusive device to facilitate sealing the neck of an aneurysm. For example, U.S. Patent No. 5,749,894 discloses vaso-occlusive devices having a polymeric coating that may be melted to seal the neck of an aneurysm within which the device is deployed. The disclosed method for melting the coating, however, requires introducing an energy source, e.g., a light-emitting device, or a radio frequency ("RF") electrical energy source, into the blood vessel adjacent the aneurysm to deliver energy to heat and melt the coating.

Further examples are found in U.S. Patent Nos. 5,853,418 and U.S. Patent Publication No. 2002/0138134. U.S. Patent No. 5,853,418 discloses a stretch-resistance member for a vaso-oclussive coil that may optionally contain a modest amount of iron. U.S. Patent Publication No. 2002/0138134 discloses a vaso-occlusive coil that can generate heat in the presence of an external magnetic field, while having an electrical connection to the exterior.

### SUMMARY

In accordance with one aspect of the invention, a vaso-occlusive device according to claim 1 is provided. By way of non-limiting example, in one embodiment, the occlusive device is provided with a highly resistive ferrous material, which is heated by a pulsed magnetic field applied by an external magnetic resonance imaging ("MRI") machine. For example, the ferrous material may be embedded or otherwise carried in or by a vaso-occlusive coil, or a coating thereon.

Heating of the occlusive device may be performed in the prior art as an end in itself, i.e., to enhance the formation of a blood thrombus embolism in the cavity, or to improve the long term healing response of the thrombus and/or surrounding aneurismal tissue, after the device is implanted. The heating of the device may also cause the device, or portions thereof, to at least partially melt and fuse together to stabilize the vaso-occlusive device in a three-dimensional (delivery) shape in the cavity. In the present invention the increase in device temperature causes a coating on the device to at least partially melt or soften, thereby releasing or otherwise activating a therapeutic or diagnostic agent within the cavity.

### BRIEF DESCRIPTION OF THE DRAWINGS

The drawings illustrate the design and utility of embodiments of the invention, in which similar elements are referred to by common reference numerals, and in which:
FIG. 1A is a schematic view of a system for embolizing a cavity within a patient's body, in accordance with one embodiment of the invention.
FIG. 1B is a cross-sectional detail of the patient's body, showing a vaso-occlusive device being delivered into an aneurysm.
FIGS. 2A and 2B are side views of an exemplary embodiment of a vaso-occlusive device constructed in accordance with the invention.
FIGS. 3A-3C are cross-sectional views of a patient's body, illustrating a method for treating an aneurysm performed with a device of the invention.
FIGS. 4A and 4B are partial cross-sectional views of further exemplary embodiments of vaso-occlusive devices constructed in accordance with a further aspect of the invention.

### DETAILED DESCRIPTION OF THE ILLUSTRATED EMBODIMENTS

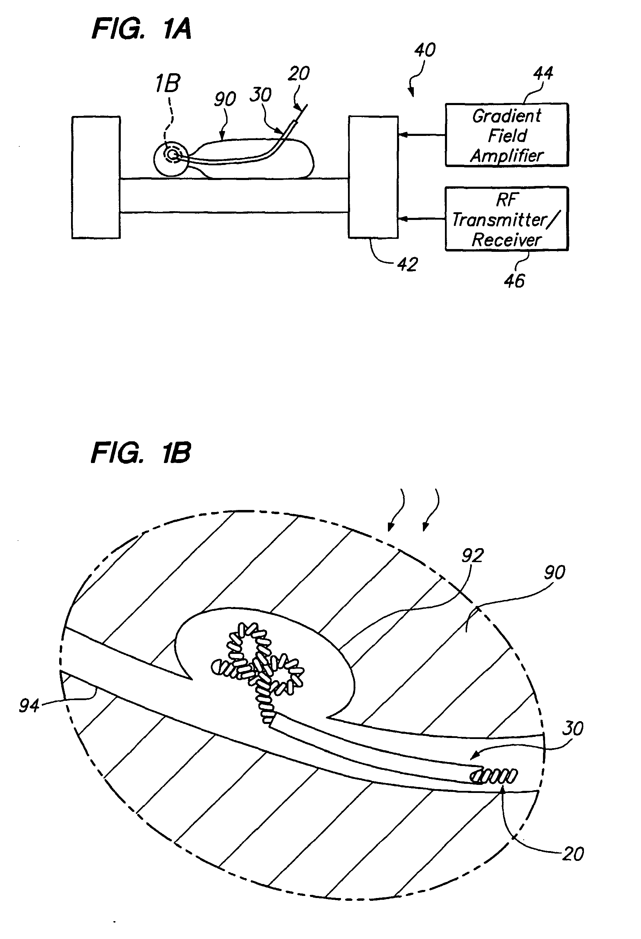

Turning to the drawings, FIGS. 1A and 1B depict a system 10 for embolizing and/or occluding a cavity within a body 90, e.g., an aneurysm 92 extending from a blood vessel 94. Generally, the system 10 includes a vaso-occlusive device 20, a delivery catheter 30, and a magnetic resonance imaging ("MRI") machine 40.

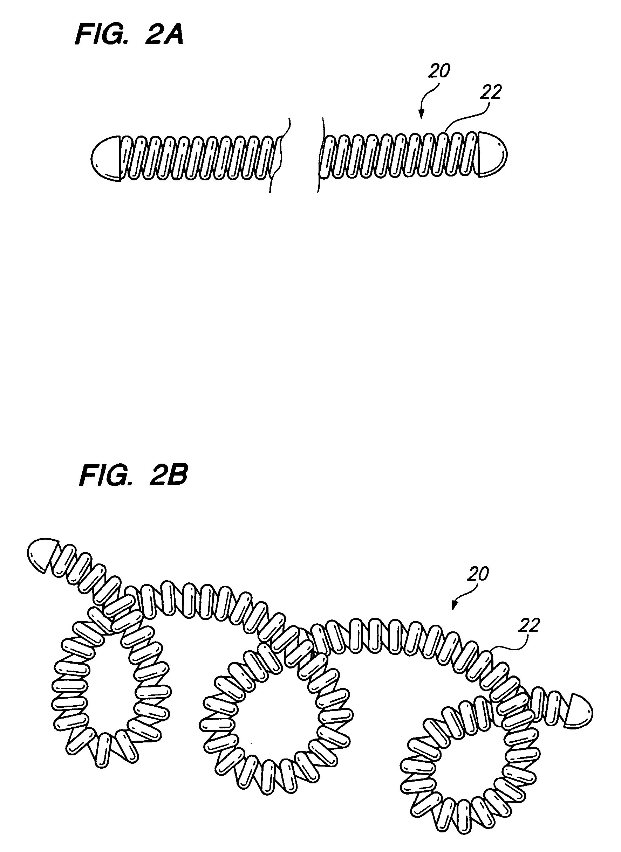

In an exemplary embodiment, such as that shown in FIGS. 2A and 2B, the vaso-occlusive device 20 includes an elongate helical coil 22 that assumes a delivery configuration (shown in FIG. 2A) when constrained in the delivery catheter 30, and a three-dimensional deployed configuration (shown in FIG. 2B) when placed in a body cavity, e.g., in aneurysm 92. The coil 22 may be formed by first winding a small, flexible wire into a linear helical form defining the delivery configuration, using known heat treatment methods. Once the delivery shape is set, the coil 22 is wound into a deployed configuration, e.g., by winding the coil 22 onto a mandrel (not shown) and heat treating the coil 22, using known methods. Thus, when the coil 22 is free from external forces, it assumes a relaxed, three-dimensional deployed configuration, as shown in FIG. 2B. It will be appreciated by those skilled in the art that the coil 22 may be biased to assume a variety of predetermined or random shapes in the deployed configuration. Alternatively, a substantially straight wire or filament, or braid of wires or filaments may be provided instead of the helical coil configuration. Additional information on methods for manufacturing vaso-occlusive devices suitable for constructing embodiments of the present invention may be found in U.S. Patent No. 6,322,576 to Wallace et al..

The coil 22 may be formed from a variety of materials, e.g., metals, polymers, alloys, or composites thereof. In one embodiment of the invention, described in greater detail blow, the coil 22 includes ferrous material, causing the coil 22 to be heated by activation of the MRI machine to apply a pulsed magnetic field on the device after it is implanted at a selected occlusion site in the vasculature.

In addition, the coil 22 may include radiopaque material(s), such as metals and/or polymers. Suitable metals and alloys for the wire defining the coil 22 may include the platinum group metals, especially platinum, rhodium, palladium, and rhenium, as well as tungsten, gold, silver, tantalum, and alloys of these metals. These metals have significant radiopacity and their alloys may be tailored to accomplish an appropriate blend of flexibility and stiffness for the coil 22. They are also generally biologically inert. By way of non-limiting example, a platinum/tungsten alloy is suitable, with ferrous material mixed with or carried by the alloy. Additional suitable materials are described in the above-referenced U.S. Patent No. 6,322,576.

Additionally or alternatively, the coil 22 may include radiolucent fibers or polymers (or metallic threads coated with radiolucent or radiopaque fibers), such as Dacron (polyester), polyglycolic acid, polylactic acid, fluoropolymers (polytetrafluoroethylene), Nylon^{™} (polyamide), and/or silk. When a polymer is used as the major component of the vaso-occlusive device 20, it may be filled with some amount of radiopaque material, such as powdered tantalum, tungsten, bismuth oxide, barium sulfate, and the like. In addition, the ferrous material, e.g., iron particles, filaments and the like, may be mixed with and/or embedded in the polymer.

When the coil 22 is made from a platinum alloy or superelastic alloy, such as nitinol, or other materials, the diameter of the wire defining the coil 22 may be between about 0.0005 and 0.006 inch (0.012-0.15 mm). The wire may be wound into a primary coil having a primary diameter between about 0.005 and 0.025 inch (0.125-0.625 mm), and preferably between about 0.010 and 0.018 inch (0.25-0.45 mm). Such wire may be of an appropriate diameter to provide sufficient hoop strength to hold the vaso-occlusive device 20 in place within a chosen body cavity without distending the wall of the cavity and/or without moving substantially from the cavity as a result of the repetitive fluid pulsing experienced within the vascular system.

The axial length of the delivery configuration of the coil 22 may be between about one half and one hundred centimeters (0.5-100 cm), and preferably between about two and forty centimeters (2-40 cm). In the delivery configuration, the coil 22 may have between about ten and seventy five (10-75) turns per centimeter, and preferably between about ten and forty (10-40) turns per centimeter.

In one embodiment, a coating is provided on the coil 22, the coating having a melting temperature "Tₘ" or a glass transition temperature "T_{g}" that is less than the temperature to which the vaso-occlusive device 20 is heated by the MRI machine 40. For example, the coating may have a "Tₘ" and/or "T_{g}" of about 26.66°C (80°F) - 66.55°C (150°F). Suitable polymeric materials may include polyalkenes, polymethacrylates, polyacrylates, polyesters, polyamides, and polysaccharides. Co-polymers, blends, alloys, and block copolymers of such materials may also be used. Additional information on suitable coatings are described in the above-referenced U.S. Patent Nos. 5,749,894 and 6,187,024.

The coating comprises, or otherwise covers, one or more agents, e.g., a bioactive agent, a collagenous material, and/or other diagnostic or therapeutic agent(s). Exemplary bioactive agents may include genes, growth factors, biomolecules, peptides, oligonucleotides, members of the integrin family, RGD-containing sequences, oligopeptides, fibronectin, laminin, bitronectin, hyaluronic acid, silk-elastin, elastin, fibrinogen, and other basement membrane proteins with bioactive agents. By way of non-limiting example, the agent(s) may be applied in a first coating on the coil 22, with a second coating, such as the polymeric materials described above, applied to substantially cover or otherwise embed the agent(s) from exposure to the blood pool and body tissue at the deployment site in the vasculature. Alternatively, the vaso-occlusive device 20 (e.g., coil 22) may itself be formed from a polymeric material having with one or more embedded diagnostic and/or therapeutic agents that are released by heating of the device, as described in greater detail below.

In particular, one or more agent(s) may be carried on, or otherwise embedded in, the vaso-occlusive device 20 without being initially exposed or otherwise released or activated in the body upon implantation of the device. This can be advantageous, e.g., for preventing the agent(s) from release or activation if the vaso-occlusive device 20 is exposed within a blood vessel or other body region where embolization or other treatment effects are undesired. Only when the coating and/or the coil itself is heated above its melting and/or glass transition temperature, e.g., when it is determined that the implant device is placed where desired in the body cavity, are the agent(s) released or otherwise activated. For example, the agents may be prevented from making contact with the blood/tissue in the body cavity until released by the heating of the implant device (as described above). Additionally or alternatively, the agents may be exposed to the blood /tissue in the cavity, but dormant until heated (i.e., by conductive heating from the heated ferrous material) above a critical temperature threshold, activating (with the heat as the catalyst) the bioactive agent.

Returning to FIG. 1A, the MRI machine 40 includes a static field magnet 42, a gradient field amplifier 44 and a radio frequency ("RF") transmitter/receiver 46. The magnet 42 includes an internal lumen region for receiving the patient 90, and may provide a static, relatively homogeneous magnetic field over the patient 90. Alternatively, an open-MRI machine or other MR device may be used, as is well-known. During conventional operation, the gradient field amplifier 44 generates pulsed magnetic field gradients that vary the static magnetic field, and the RF transmitter 46 transmits RF pulse sequences over the patient's body to cause the patient's tissues to emit MR response signals. The MR response signals may be used to generate images of the patient's body 90, as is well-known.

When the MRI machine 40 is activated, the pulsed magnetic field generated by the magnet 42 and gradient field amplifier 44 will agitate the ferrous material in the vaso-occlusive device 20, thereby causing the ferrous material to heat. Other portions of the vaso-occlusive device 20, e.g., a coating and/or polymeric material defining the coil 22 itself, are heated by conduction from the heated ferrous material above their "Tₘ" and/or "T_{g}," causing the coating and/or polymeric material portions to at least partially melt or soften. Additionally or alternatively, heat generated by the interaction between the MRI device 40 and the ferrous material in the vaso-occlusive device 20 heats the surrounding blood or tissue to enhance embolization of the site and/or promote improved long term healing of the embolism and/or surrounding aneurysmal tissue. Notably, a coating on the occlusive device may itself include ferrous material.

More particularly, the pulsed magnetic field can heat the ferrous material through at least two mechanisms. First, the material can be heated due to hysteresis losses associated with the material being alternatively energized with positive and negative energy fields; i.e., as some materials are less efficient in responding to the alternating polarization, resulting in current losses which produce heat. Materials that are poor conductors, such as ferrous materials, produce more heat effects than highly conductive materials such as copper, platinum or aluminum. Other factors that effect hysteresis losses include the flux density and the MR frequency of the selected materials.

A second mechanism for the generation of heat involves conduction losses. The MR machine produces a conduction field around the device, thereby producing a voltage potential across the device. This voltage potential produces eddy currents in the occlusive device. Some materials and some implant device constructions are more conductive than others. Less conductive implants will result in more heat being generated. By way of example, flat ribbon material, rather than round wire stock, will increase resistance (and, thus, heating), as will using ferrous materials over conductive materials such as copper, platinum or aluminum.

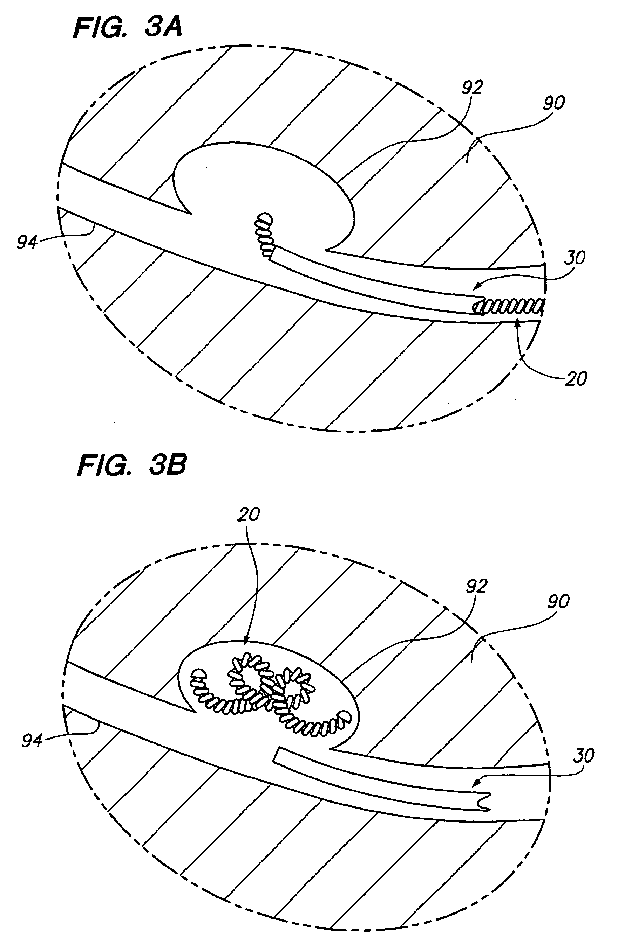

Turning to FIGS. 3A-3C, a method for embolizing and/or occluding an aneurysm 92 is illustrated that employs the system 10 shown in FIG. 1A. In this exemplary method, the vaso-occlusive device 20 includes a bioactive agent, collagenous material, and/or other therapeutic and/or diagnostic agent embedded within or beneath a coating on the coil 22.

Initially, the delivery catheter 30 is introduced into the patient's body 90 from a percutaneous entry site into a peripheral artery, such as the femoral or carotid arteries (not shown), as is well-known. The delivery catheter 30 may be advanced over a guidewire or other rail (not shown) previously placed within the patient's vasculature using known methods. The delivery catheter 30 is advanced through the patient's vasculature, until a distal end 32 of the catheter 30 is disposed within a blood vessel 94 adjacent the aneurysm 92.

Once the catheter 30 is properly positioned, the vaso-occlusive device 20 is advanced through a lumen 34 of the catheter 30 into the aneurysm 92, as shown in FIG. 3A. As the vaso-occlusive device 20 is deployed in the aneurysm, it assume a three-dimensional, deployed configuration. The deployed configuration is generally selected so that the vaso-occlusive device 20 substantially fills the aneurysm 92. Once the vaso-occlusive device 20 is fully deployed within the aneurysm 92, as shown in FIG. 3B, the delivery catheter 30 is removed. Depending on the circumstances (e.g., size and condition of the aneurysm), a treating physician will deploy multiple occlusive devices into a single aneurysm, as is well-known. Additional information on apparatus and methods that may be suitable for delivering the vaso-occlusive coil 20 is found in U.S. Patent No. 4,994,069 to Ritchart et al., as well as in the above-referenced U.S. patents.

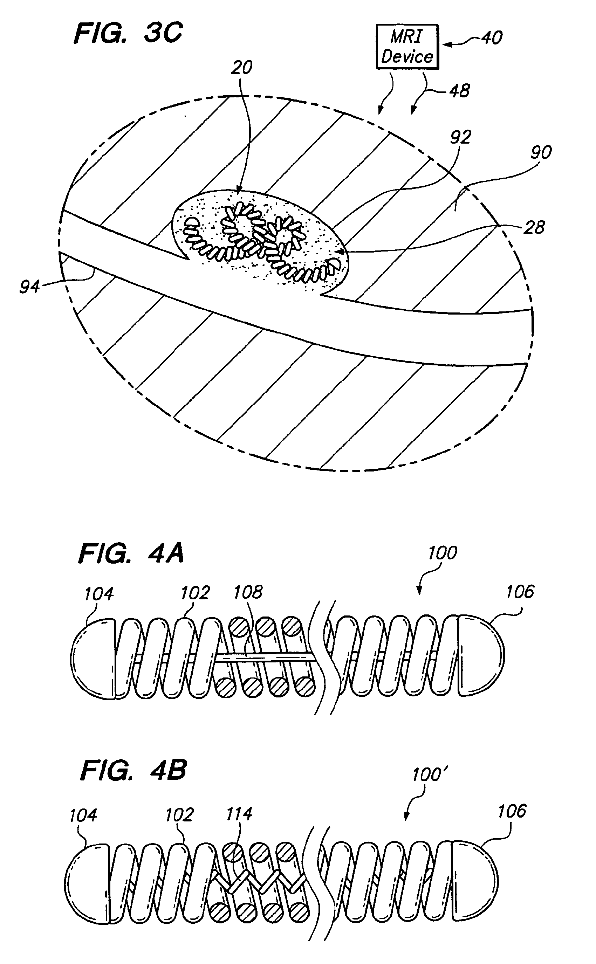

Turning to FIG. 3C, once the occlusive coil 22 is implanted and the delivery catheter 30 removed from the body, the MRI machine 40 is activated to at least partially melt or sufficiently soften the coating on the coil 22. This may involve transferring the patient 90 from a catheter lab or other location where the vaso-occlusive device 20 was implanted into a MRI room. As explained above, once the patient 90 is disposed within the MRI magnet 42, the gradient field amplifier 44 (not shown in FIG. 3C, see FIG. 1A) is activated to generate magnetic energy (represented by arrows 48 in FIG. 3C), which interacts with ferrous material in the coil 22 and/or coating to heat the vaso-occlusive device 20. Generally, the MRI machine 40 is activated for a predetermined time, e.g., between about 3 seconds to 20 minutes, to heat the vaso-occlusive device 20 and/or coating to a desired temperature. For example, the vaso-occlusive device and/or coating may be heated to a temperature of at least about 40 °C (about 150° F).

As the coating reaches its "Tₘ" and/or "T_{g}," the coating may melt and/or flow, thereby releasing or otherwise activating one or more diagnostic or therapeutic agent(s) 28 carried in or beneath the coating. For example, the coating may include a thrombogenic agent that enhances embolization of blood when released within the aneurysm 92. In addition or alternatively, the coating may include one or more agents that remain substantially inert until heated above a predetermined activation temperature. Further additionally or alternatively, the MRI machine 40 may be activated for the to heat the vaso-occlusive device 20 in order to heat blood or other material within the aneurysm 92 and/or tissue surrounding the aneurysm 92, even in the absence of such agent(s). Such heating may accelerate coagulation of blood or other fluid within the aneurysm 92 and/or may cause the surrounding tissue to contract, e.g., to reduce the size of the aneurysm 92, and promote an improved long term healing response.

In accordance with a further aspect of the invention, in an alternate embodiment, at least a portion of the vaso-occlusive device 20 is formed from a material that melts or is otherwise sufficiently softened when heated. In this embodiment, the MRI machine 40 is activated for sufficient time to cause at least a portion of a coating and/or coil of the vaso-occlusive device 20 to melt and/or flow together. When the MRI machine 40 is deactivated, the coil cools, and substantially solidifies, thereby fusing together melted/softened portions of the vaso-occlusive device 20 in its deployed configuration in the aneurysm 92. This fusion stabilizes the vaso-occlusive device 20 in its deployed configuration, helping prevent the device from moving back towards a delivery and/or other generally linear shape.

FIGS. 4A and 4B are partial cross-sections (or cutaways) of further embodiments of MR-activated, embolic implant devices 100 and 100', respectively, constructed in accordance with a further aspect of the present invention. The devices 100 and 100' are each made up of a helically wound occlusive coil 102 having a first end 104 and a second end 106. While the coil 102 is depicted in FIGS. 4A and 4B as made from a round wire, i.e., having a substantially circular cross-section, the coil 102 may alternatively be made from a differently shaped wire, e.g., a flat wire having a rectangular cross-section. The coil 102 may be made of platinum, and may be provided with a heat-releasable and/or activated agent coating (not shown), as discussed above. Additionally or alternately, a heat-releasable and/or activated agent coating is provided on the filament 108, 114.

The coil 102 forms a central lumen, through which a ferrous material filament 108 (FIG. 4A), 114(FIG. 4B) is located, the filament being fixed to the respective first and second ends 104 and 106 of the coil 102. In this manner, the filament 108, 114 acts as a heating element upon application of an AC magnetic field by an MRI machine, as discussed above. In particular, the highly conductive (e.g., platinum) coil 102 generates a corresponding highly efficient induction field, which heats the highly resistive ferrous material filament 108, 114 in the coil lumen. Thermal energy in the heated filament is then transferred by convection to the coil 102 and surrounding blood pool and tissue, which has the benefits/effects previously described.

Further additionally or alternately, at least a portion of the coil 102 may be formed from a material that melts or substantially softens when heated, as described above. In this case, fusing points of the coil 102 in its deployed configuration may be determined by locations that the ferrous filament is attached, since these attachment points on the coil 102 will heat the most. It will be appreciated from the present disclosure that the features of a heat-releasable and/or activated agent and structural fusing of the occlusive coil may be provided separately or together in a single, implantable device.

The filament 108, 114 may also (optionally) serve as a stretch resisting member, as taught, for example, in U.S. Patent 5,853,418. In certain circumstances, it may be desirable to attach the filament 108, 114 only to one of the two ends 104, 106, or alternately (or additionally) to at least one site between the to ends, or to neither of the two ends. Of course, for attaining stretch resistance, if so desired, the filament 108, 114 must be attached to at least two points on the coil 102.

The ferrous filament 108, 114 may be constructed in various ways. For example, the filament 108, 114 may comprise thermoplastic or thermosetting having a bundle of threads or a single filament with one or more metallic ferrous strands formed therein. The filament 108 of the variation shown in FIG. 4A is formed as a ribbon; the filament 114 shown in FIG. 4B is formed as a helically wound coil; in both cases that is soldered, brazed, glued, or otherwise fixedly attached to the first and second coil ends 104 and 106.

In further alternative embodiments, other devices or processes may be employed for in-situ heating of vaso-occlusive devices in accordance with the present invention. For example, an ultrasound device (not shown) may be provided, e.g., including a piezoelectric transducer that may be placed in contact with the patient's skin overlying an aneurysm, lumen, or other cavity where a vaso-occlusive device has been implanted. If desired, an acoustic gel or other material may be provided between the transducer and the patient's skin to enhance acoustically coupling the transducer to the patient, as is known in the art.

Acoustic energy may be delivered from the ultrasound device through the patient's skin and intervening tissue to the cavity to heat the vaso-occlusive device. The energy may be focused or generally directed into the patient's body using known methods. The acoustic energy may be transferred to heat energy when it is absorbed by the vaso-occlusive device and/or surrounding tissue to heat the vaso-occlusive device. The vaso-occlusive device may include materials that enhance acoustic energy absorption and/or attenuation in a predetermined manner to ensure adequate heating of the vaso-occlusive device.

In further alternate embodiments, a source of electrical energy, e.g., a radio frequency ("RF") generator, may be provided adjacent the patient's skin. Electrical energy may be delivered into the patient's body to inductively heat the vaso-occlusive device, as is known to those skilled in the art.

Although the present invention has been described with reference to occlusion and treatment of vasculature sites, such as aneurysms, those skilled in the art will recognize that the methodology of heating an implanted device using an energy source outside of the body could be used for other types of conditions, such as for heating tumors, releasing bioactive agents (e.g., using a low level of heating energy) at any number of intra-body locations, as well as to heat other types of implants, e.g., stents and the like, to enhance patient treatment.

## Claims

1. A vaso-occlusive device (20, 100, 100') for treating a site within a patient's vasculature, comprising:
a first material which, if the device is implanted at a treatment site in the patient's vasculature, may be heated by application of a source of energy external to the patient;
a bioactive agent; and
**characterized in that**,
if the device is implanted at a treatment site in the patient's vasculature, the bioactive agent is released from the device into the treatment site or otherwise activated upon heating of the device by application of the external energy source to heat the first material.

2. The vaso-occlusive device (20, 100, 100') of claim 1, further comprising a second material having a melting or glass transition temperature greater than body temperature, but less than a temperature reached by the device when the first material is heated by the external energy source.

3. The vaso-occlusive device (20, 100, 100') of claim 2, wherein the second material is embedded in one or more portions of the device, such that, if the device is implanted at the treatment site when heated by the external energy source, and thereafter allowed to cool at the treatment site, the one or more portions are at least partially melted and fused together to thereby stabilize the vaso-occlusive device in a deployed configuration.

4. The vaso-occlusive device (20, 100, 100') of claim 2, the second material comprising a coating provided on at least a portion of the device.

5. The vaso-occlusive device (20, 100, 100') of claim 1, the first material comprising a ferrous material, and the external energy source comprising magnetic resonance.

6. The vaso-occlusive device (20, 100, 100') of claim 1, wherein the first material is embedded in the device.

7. The vaso-occlusive device (20, 100, 100') of claim 1, wherein the first material is in a coating provided on at least a portion of the device.

8. The vaso-occlusive device (20, 100, 100') of claim 1, the device comprising
a coil forming a lumen (108,114), and
a heating member disposed in the lumen (108, 114), the heating member at least partially comprising the first material.

9. The vaso-occlusive device (20, 100, 100') of claim 8, the heating member comprising a filament attached to first and second locations of the coil.

10. The vaso-occlusive device (20, 100, 100') of claim 8, further comprising a second material having a melting or glass transition temperature greater than body temperature, but less than a temperature reached by the heating member when the first material is heated by the external energy source.

11. The vaso-occlusive device (20, 100, 100') of claim 10, wherein the second material is embedded in one or more portions of the coil, such that, if the coil is implanted at the treatment site when heated by the heating member, and thereafter allowed to cool at the treatment site, the one or more portions are at least partially melted and fused together to thereby stabilize the coil in a deployed configuration.

12. The vaso-occlusive device (20, 100, 100') of claim 10, the second material comprising a coating provided on at least a portion of the coil.

13. The vaso-occlusive device (20, 100, 100') of claim 10, the heating member comprising a filament attached to the coil, the second material comprising a coating provided on at least a portion of the filament.

## Patentansprüche

1. Gefäßokklusionsvorrichtung (20, 100, 100') zur Behandlung einer Stelle im Gefäßsystem eines Patienten, umfassend :
ein erstes Material, das, wenn die Vorrichtung an einer Behandlungsstelle im Gefäßsystem des Patienten implantiert ist, durch Anlegen einer Energiequelle extern zu dem Patienten erhitzt werden kann ;
ein bioaktives Mittel ; und
**dadurch gekennzeichnet, dass**,
wenn die Vorrichtung an einer Behandlungsstelle im Gefäßsystem des Patienten implantiert ist, das bioaktive Mittel, beim Erhitzen der Vorrichtung durch Anlegen der externen Energiequelle zum Erhitzen des ersten Materials, von der Vorrichtung in die Behandlungsstelle freigesetzt oder anderweitig aktiviert wird.

2. Gefäßokklusionsvorrichtung (20, 100, 100') nach Anspruch 1, weiter ein zweites Material umfassend, das eine Schmelz- oder Glasübergangstemperatur aufweist, die höher als Körpertemperatur ist, jedoch niedriger als eine von der Vorrichtung, wenn das erste Material durch die externe Energiequelle erhitzt wird, erreichte Temperatur.

3. Gefäßokklusionsvorrichtung (20, 100, 100') nach Anspruch 2, wobei das zweite Material in einen oder mehrere Teile der Vorrichtung eingebettet ist, sodass, wenn die Vorrichtung an der Behandlungsstelle implantiert ist, wenn sie von der externen Energiequelle erhitzt wird, und man sie danach an der Behandlungsstelle abkühlen lässt, die einen oder mehreren Teile mindestens teilweise geschmolzen und zusammengeschmolzen werden, um **dadurch** die Gefäßokklusionsvorrichtung in einer entfalteten Konfiguration zu stabilisieren.

4. Gefäßokklusionsvorrichtung (20, 100, 100') nach Anspruch 2, wobei das zweite Material eine an mindestens einem Teil der Vorrichtung angebrachte Beschichtung umfasst.

5. Gefäßokklusionsvorrichtung (20, 100, 100') nach Anspruch 1, wobei das erste Material ein Eisenmaterial umfasst und die externe Energiequelle Magnetresonanz umfasst.

6. Gefäßokklusionsvorrichtung (20, 100, 100') nach Anspruch 1, wobei das erste Material in der Vorrichtung eingebettet ist.

7. Gefäßokklusionsvorrichtung (20, 100, 100') nach Anspruch 1, wobei das erste Material sich in einer an mindestens einem Teil der Vorrichtung angebrachten Beschichtung befindet.

8. Gefäßokklusionsvorrichtung (20, 100, 100') nach Anspruch 1, wobei die Vorrichtung umfasst
eine ein Lumen (108, 114) bildende Spirale, und
ein in dem Lumen (108, 114) angeordnetes Heizelement, wobei das Heizelement mindestens teilweise das erste Material umfasst.

9. Gefäßokklusionsvorrichtung (20, 100, 100') nach Anspruch 8, wobei das Heizelement ein an einem ersten und einem zweiten Standort der Spirale befestigtes Filament umfasst.

10. Gefäßokklusionsvorrichtung (20, 100, 100') nach Anspruch 8, weiter ein zweites Material umfassend, das eine Schmelz- oder Glasübergangstemperatur aufweist, die höher als Körpertemperatur, jedoch niedriger als eine von dem Heizelement erreichte Temperatur, wenn das erste Material von der externen Energiequelle erhitzt wird, ist.

11. Gefäßokklusionsvorrichtung (20, 100, 100') nach Anspruch 10, wobei das zweite Material in einem oder mehreren Teilen der Spirale eingebettet ist, sodass, wenn die Spirale an der Behandlungsstelle implantiert ist, wenn sie von dem Heizelement erhitzt wird, und man sie danach an der Behandlungsstelle abkühlen lässt, die einen oder mehreren Teile mindestens teilweise geschmolzen und zusammengeschmolzen werden, um **dadurch** die Gefäßokklusionsvorrichtung in einer entfalteten Konfiguration zu stabilisieren.

12. Gefäßokklusionsvorrichtung (20, 100, 100') nach Anspruch 10, wobei das zweite Material eine an mindestens einem Teil der Spirale angebrachte Beschichtung umfasst.

13. Gefäßokklusionsvorrichtung (20, 100, 100') nach Anspruch 10, wobei das Heizelement ein an der Spule befestigtes Filament umfasst, wobei das zweite Material eine an mindestens einem Teil des Filaments angebrachte Beschichtung umfasst.

## Revendications

1. Dispositif vaso-occlusif (20, 100, 100') pour traiter un site au sein du système vasculaire d'un patient, comprenant :
une première matière qui, lorsque le dispositif est implanté à un site de traitement dans le système vasculaire d'un patient, peut être chauffée par application d'une source d'énergie externe au patient ;
un agent bioactif ; et
**caractérisé en ce que**
lorsque le dispositif est implanté à un site dans le système vasculaire du patient, l'agent bioactif est libéré du dispositif pour pénétrer dans le site de traitement ou bien est activé d'une autre manière lorsqu'on chauffe le dispositif par l'application de la source d'énergie externe afin de chauffer la première matière.

2. Dispositif vaso-occlusif (20, 100, 100') selon la revendication 1, comprenant une deuxième matière possédant une température de fusion ou de transition vitreuse supérieure à la température du corps, mais inférieure à une température qu'atteint le dispositif lorsque la première matière est chauffée par la source d'énergie externe.

3. Dispositif vaso-occlusif (20, 100, 100') selon la revendication 2, dans lequel la deuxième matière est incluse dans une ou plusieurs portions du dispositif, de telle sorte que, lorsque le dispositif est implanté au site de traitement alors qu'il est chauffé par la source d'énergie externe, et lorsqu'on le laisse refroidir par la suite au site de traitement, lesdites une ou plusieurs portions sont portées au moins en partie à fusion et fusionnent réciproquement pour ainsi stabiliser le dispositif vaso-occlusif à l'état déployé.

4. Dispositif vaso-occlusif (20, 100, 100') selon la revendication 2, dans lequel la deuxième matière comprend un revêtement recouvrant au moins une portion du dispositif.

5. Dispositif vaso-occlusif (20, 100, 100') selon la revendication 1, 1a première matière comprend une matière ferreuse et la source d'énergie externe comprend une résonance magnétique.

6. Dispositif vaso-occlusif (20, 100, 100') selon la revendication 1, dans lequel la première matière est incluse dans le dispositif.

7. Dispositif vaso-occlusif (20, 100, 100') selon la revendication 1, la première matière est présente dans un revêtement recouvrant au moins une portion du dispositif.

8. Dispositif vaso-occlusif (20, 100, 100') selon la revendication 1, le dispositif comprenant :
une spirale formant une lumière (108, 114) ; et
un membre chauffant disposé dans la lumière (108, 114), le membre chauffant comprenant au moins en partie la première matière.

9. Dispositif vaso-occlusif (20, 100, 100') selon la revendication 8, dans lequel le membre chauffant comprend un filament fixé à un premier et à un deuxième endroit de la spirale.

10. Dispositif vaso-occlusif (20, 100, 100') selon la revendication 8, comprenant en outre une deuxième matière possédant une température de fusion ou de transition vitreuse supérieure à la température du corps, mais inférieure à une température qu'atteint le dispositif lorsque la première matière est chauffée par la source d'énergie externe.

11. Dispositif vaso-occlusif (20, 100, 100') selon la revendication 10, dans lequel la deuxième matière est incluse dans une ou plusieurs portions de la spirale, de telle sorte que, lorsque la spirale est implantée au site de traitement alors qu'elle est chauffée par la source d'énergie externe, et lorsqu'on la laisse refroidir par la suite au site de traitement, lesdites une ou plusieurs portions sont portées au moins en partie à fusion et fusionnent réciproquement pour ainsi stabiliser la spirale à l'état déployé.

12. Dispositif vaso-occlusif (20, 100, 100') selon la revendication 10, dans lequel la deuxième matière comprend un revêtement recouvrant au moins une portion de la spirale.

13. Dispositif vaso-occlusif (20, 100, 100') selon la revendication 10, dans lequel le membre chauffant comprend un filament fixé à la spirale, la deuxième matière comprenant un revêtement recouvrant au moins une portion du filament.
